# EUROPEAN PATENT SPECIFICATION

(11) **EP 3 096 694 B1**
(45) Date of publication and mention of the grant of the patent: **01.01.2020**
(21) Application number: 15740445.0
(22) Date of filing: 21.01.2015
(51) Int. Cl.: A61B 17/15

(54) **SKIN-REFERENCING SURGICAL GUIDES**
HAUTBEZOGENE CHIRURGISCHE FÜHRUNGEN
GUIDES CHIRURGICAUX DE RÉFÉRENCEMENT DE LA PEAU

(30) Priority: 23.01.2014 US 201461930878 P
(43) Date of publication of application: 30.11.2016
(73) Proprietor: ConforMIS, Inc., Billerica, MA 01821 (US)
(72) Inventor: BOJARSKI, Raymond, A., Attleboro, MA 02703 (US); KURTZ, William, B., Nashville, TN 37205 (US)
(74) Representative: Grund, Martin
(86) International application number: PCT/US2015/012203
(87) International publication number: WO 2015/112570

(56) References cited:
- US-A1- 2007 288 030
- US-A1- 2012 053 594
- US-A1- 2012 209 276
- US-A1- 2013 184 713
- US-A1- 2013 184 713
- US-A1- 2013 184 764
- US-A1- 2013 331 850

## Description

### FIELD

The present teachings generally relate to surgical repair systems (e.g., resection cut strategy, guide tools, and implant components) as described in, for example, U.S. Patent Application Serial No. 13/397,457, entitled "Patient-Adapted and Improved Orthopedic Implants, Designs And Related Tools," filed February 15, 2012, and published as U.S. Patent Publication No. 2012-0209394. In particular, the present teachings provide surgical tools, systems, methods, and techniques incorporating features to facilitate preparation of a patient's anatomical surfaces for installation of implant components.

The invention relates to guides and systems as defined in claims 1 and 7, preferred embodiments of the invention are defined in the dependent claims.

### BACKGROUND

The natural anatomical joint structures of an individual may undergo degenerative changes due to a variety of reasons, including injury, osteoarthritis, rheumatoid arthritis, or post-traumatic arthritis. When such damage or degenerative changes become far advanced and/or irreversible, it may ultimately become necessary to replace all or a portion of the native joint structures with prosthetic joint components. Joint replacement is a well-tolerated surgical procedure that can help relieve pain and restore function in injured and/or severely diseased joints, and a wide variety of prosthetic joints are well known in the art, with different types and shapes of joint replacement components commercially available to treat a wide variety of joint conditions.

As part of the surgical repair procedure, the underlying anatomical support structures are typically prepared to receive the joint implant components. For example, the placement of a femoral implant component can typically involve preparation of the caudad portion of the femoral bone (otherwise known as the distal head of the femur). This may include surgical resection (e.g., cutting, drilling, rongeuring, scraping) of portions of the medial and/or lateral condyles of the femur, as well as the resection of other anatomical features of the femur and/or surrounding soft tissues. This preparation will desirably create an anatomical support structure capable of accommodating and adequately supporting the femoral implant component or components, which is ultimately secured to the femur. Similar surgical steps can be performed to the tibia and/or the patella, as well as other anatomical structures, as necessary.

US 2012/209276 A1 discloses a surgical cutting guide for treating a knee of a patient, the guide comprising a first portion configured for abutting against the skin of the patient and a second portion configured to conform to the surface of a tibia of a specific patient.

One or more surgical guide tools or jigs can be used to assist the surgeon in preparing the underlying anatomical support structure(s). There is a need, however, for improved surgical guide tools and jigs to improve the accuracy, reproducibility, and/or ease of preparing underlying anatomical support structure(s) for an implant and to minimize the invasiveness of such procedures.

### BRIEF DESCRIPTION OF THE DRAWINGS

FIGs. 1A and 1B are perspective views of an exemplary tibial guide tool;
FIGs. 2 and 3 are perspective views of a tibial guide tool positioned with respect to a tibia;
FIG. 4 is a perspective views of a tibial guide tool according to the invention positioned with respect to a tibia and a skin surface;
FIGs. 5A and 5B are perspective views of a skin-referencing guide tool;
FIG. 6 is a cross-sectional image of a tibia and surrounding tissue;
FIG. 7 is a perspective views of a skin-referencing guide tool positioned with respect to a tibia and a skin surface;
FIGs. 8A and 8B are perspective views of a tibial guide tool and alignment component positioned with respect to a tibia
FIG. 9 depicts perspective views of various additional tibial jigs; and
FIGs. 10A and 10B are perspective views of a removable adapter for connecting to the various jigs shown in FIG. 9.

### DETAILED DESCRIPTION

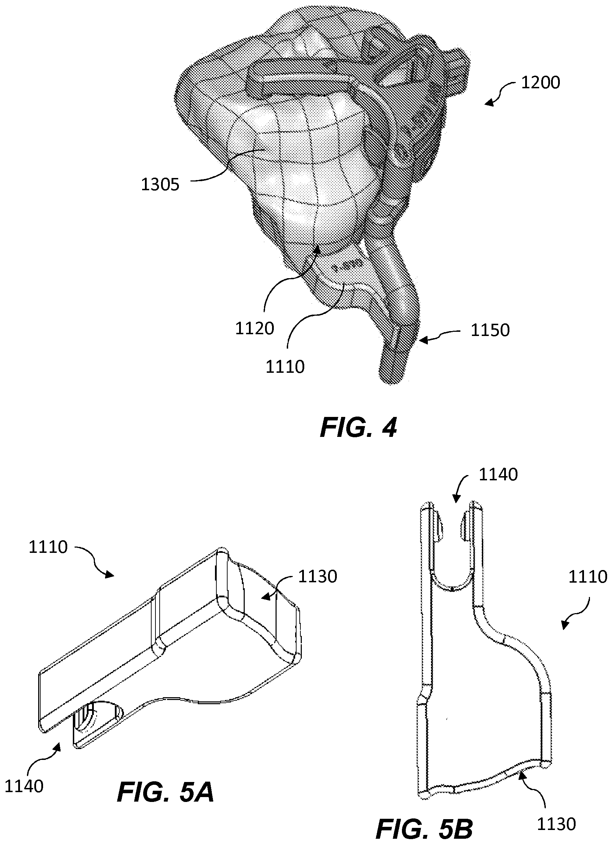

The only figure that shows a guide comprising all the features of the claimed invention is figure 4.

Wherever possible, the same reference numbers will be used throughout the drawings to refer to the same or like parts.

In this application, the use of the singular includes the plural unless specifically stated otherwise. Furthermore, the use of the term "including," as well as other forms, such as "includes" and "included," is not limiting. Also, terms such as "element" or "component" encompass both elements and components comprising one unit and elements and components that comprise more than one subunit, unless specifically stated otherwise. Also, the use of the term "portion" may include part of a moiety or the entire moiety.

The section headings used herein are for organizational purposes only and are not to be construed as limiting the subject matter described.

A variety of traditional guide tools are available to assist surgeons in preparing a joint for an implant, for example, for resectioning one or more of a patient's biological structures during a joint implant procedure. However, these traditional guide tools typically are not designed to match the shape (contour) of a particular patient's biological structure(s). Moreover, these traditional guide tools typically are not designed to impart patient-optimized placement for the resection cuts. Thus, using and properly aligning traditional guide tools, as well as properly aligning a patient's limb (e.g., in rotational alignment, in varus or valgus alignment, or alignment in another dimension) in order to orient these traditional guide tools, can be an imprecise and complicated part of the implant procedure.

Also described herein is the use of a guide tool having at least one patient-adapted bone-facing surface portion that substantially negatively-matches at least a portion of a biological surface at the patient's joint. For example, as part of the surgical planning phase, patient-specific information (as well as modeling data associated therewith, etc.) can be utilized to create one or more surgical guide tools that can assist the surgeon in preparing the underlying anatomical support structure(s). Accordingly, the guide tool(s) can include registration features that correspond to various anatomical features of the patient's anatomy and, when properly oriented relative to the anatomy, provide one or more guides that the surgeon can follow to create a desired resection (e.g., cutting, drilling, rongeuring, scraping) path in the patient's anatomy. Additionally or alternatively, certain guide tools can be used for purposes other than guiding a drill or cutting tool. For example, balancing and trial guide tools can be used to assess knee alignment and/or fit of one or more implant components or inserts. Similarly, alignment guide tools can provide anatomical registration and be utilized with a linkage to align another surgical tool or guide in a predetermined position and/or orientation based on the registration.

The use of patient-adapted (i.e., patient-specific and/or patient-engineered) jigs and associated surgical tools can provide a significant improvement in the surgical replacement of joints, and can greatly simplify the surgical procedure. By utilizing pre-operative image and/or other available data to plan the surgical procedure, a significant amount of surgical "guess-work" can be removed from the procedure. Moreover, the creation of alternative surgical plans, and the associated components to execute such alternative plans, can further facilitate the surgical execution of the procedure, allowing the surgeon to modify the procedure intraoperatively in one or more desired manners, yet significantly reducing the potential for surgical error.

Various examples of guide tools disclosed herein can include at least one patient-adapted bone-facing surface that substantially negatively-matches at least a portion of a biological surface at and/or adjacent to the patient's joint. The patient's biological surface can include cartilage, bone, tendon, skin, and/or other biological surfaces. For example patient-specific data such as imaging data of a patient's joint can be used to model an area on the articular surface. A guide tool can be selected and/or designed to have one or multiple areas that substantially negatively-match one or multiple areas on the modeled articular surface.

In various examples described herein, one or more models of at least a portion of a patient's joint can be generated. Specifically, various patient-specific data and/or measurements can be used to generate a model that includes at least a portion of the patient's joint, and in some examples, at least a portion of tissue adjacent to the joint. Various methods to generate such models can be employed, including, for example, those described in U.S. Patent Publication No. 2012-0209394. In some examples, such methods of generating a model of a patient's joint (and/or a resection cut, drill hole, guide tool, and/or implant component) or other biological feature (and/or a patient-adapted feature of a guide tool or implant component) can include the general steps of obtaining image data of a patient's biological feature; segmenting the image data; combining the segmented data; and presenting the data as part of a model. In various examples, this model can include irregular or unusual anatomical features or portions of the joint, which can include, without limitation, osteophytes, subchondral cysts, geodes or areas of eburnation, joint flattening, contour irregularity, and loss of normal shape. The model surface(s) or structure(s) can be or reflect any surface or structure in the joint and/or adjacent to the joint, including, without limitation, bone surfaces, ridges, plateaus, cartilage surfaces, ligament surfaces, skin surfaces, or other surfaces or structures.

As part of this process, one or more patient-adapted resection cuts, drill holes, guide tools, and/or implant components can be included in a model. In certain examples, a model of at least part of a patient's joint can be used to directly generate a patient-adapted resection cut strategy, a patient-adapted guide tool design, and/or a patient-adapted implant component design for a surgical procedure (i.e., without the model itself including one or more resection cuts, one or more drill holes, one or more guide tools, and/or one or more implant components). In certain examples, the model that includes at least a portion of the patient's joint also can include or display, as part of the model, one or more resection cuts and/or drill holes (e.g., on a model of the patient's tibia), one or more guide tools, and/or one or more implant components that have been designed for the particular patient using the model. Moreover, one or more resection cuts, one or more drill holes, one or more guide tools, and/or one or more implant components can be modeled and selected and/or designed separate from a model of a particular patient's biological features.

As discussed above, various examples of guide tools can include at least one guiding formation for directing movement of a surgical instrument, for example, a securing pin or a cutting tool. One or more of the guiding formations can be designed to guide the surgical instrument to deliver a patient-optimized placement for, for example, a securing pin or resection cut. In addition or alternatively, one or more of the guiding formations can be designed to guide the surgical instrument to deliver a standard placement for, for example, a securing pin or resection cut. As used herein, the terms "jig" and "guide" also can refer to guide tools. Guiding formations can comprise a variety of structures, such as, for example, surfaces, slots, holes, apertures, shielding elements, stops (e.g., depth stops), and/or any other structures intended to direct and/or limit movement of a surgical instrument. The term "slot" will be used herein to generally identify a captured guiding formation, which can have a variety of cross-sectional shapes (e.g., circular, square, rectangle, oblong, elliptical, U-shaped) and which can be configured to receive a variety of different types of surgical instruments (e.g., drill, saw, broach, pins, K-wires). The slots in a particular guide tool can be, for example, substantially horizontal, substantially diagonal, or substantially vertical as compared to the patient's mechanical axis and/or anatomical axis. Moreover, one or more of the resection cut slots can allow for a complete resection cut or a partial resection cut, e.g., scoring of the patient's bone to establish a resection cut that can be finished after removing the tool.

The various guide tools described herein can include any combination of patient-adapted features and/or standard features. For example, a patient-adapted guide tool can include at least one feature that is preoperatively designed and/or selected to substantially match one or more of the patient's biological features. A patient-engineered guide tool can include at least one feature that is designed or selected based on patient-specific data to optimize one or more of the patient's biological features to meet one or more parameters. A standard guide tool can include at least one feature that is selected from among a family of limited options, for example, selected from among a family of 5, 6, 7, 8, 9, or 10 options. Moreover, in certain examples a set or kit of guide tools can be provided in which certain guide tools in the set or kit include patient-specific, patient-engineered, and/or standard features.

In various exemplary examples, a series of guide tools can be designed, selected and/or modified to assist a surgeon in preparing a patient's tibia and/or other anatomical structure(s) to provide sufficient and proper anatomical support for one or more tibial implant components. For example, various examples can include one or more guide tools designed to guide the surgeon in performing one or more patient-adapted cuts to the bone so that those cut bone surface(s) negatively-match and/or are otherwise appropriate to bone cuts and/or other features of the implant component. Various sets of guide tools described herein can be designed for a "tibia-first" and/or a "femur-first" cut technique, although various other bones (such as the femur and/or patella, for example) can be prepared as part of and/or independently of the tibia-related procedures. In various examples, the tibial implant component suitable for use with such tools can comprise patient-adapted (i.e., patient-specific and/or patient-engineered) features, as well as standard (i.e., non-patient-specific) features. While the designs, procedures and tools are disclosed and described in combination with a patient-adapted implant component, the present disclosure may be employed with varying utility to prepare a patient's tibia to provide sufficient and proper anatomical support for standard and/or modular tibial implant components in a similar manner.

In various examples, a first tibial guide tool or jig can include features that correspond to specific anatomical features of a patient's anatomy, and this first jig can be aligned with the patient anatomy and used to prepare a tibial bone for a surgical procedure. In some examples, in a first step, the first jig can be used to identify soft tissue structures (e.g., articular cartilage structures) and/or other anatomical structures to be removed, modified and/or otherwise accommodated in some manner to receive a substantially matching or conforming surface of the first jig, which can then be used to establish peg holes and/or pin placements (or other known reference positions) to secure, guide and/or align various guide tools or jigs used to prepare the tibia for one or more implant components. The first jig can be designed to accommodate varying cartilage thickness, if desired, or the jig can be used to identify and/or verify the removal of interfering structures such as articular cartilage and/or osteophytes that may exist on relevant portions of the tibia. The first jig can include an inner surface that substantially conforms to or otherwise accommodates relevant and accessible portions of the outer surface of an uncut tibia, which may include surface features of the articular cartilage, osteophytes and/or other surface features, subchondral bone (e.g., where articular cartilage has been worn, degraded or previously removed by the surgeon) and/or one or more pre-existing implant components and/or cut surfaces (e.g., in the case of an implant revision procedure) or various combinations thereof.

In some examples the first jig can fit onto or otherwise accommodate the tibia in a predetermined position and/or orientation. Optionally, the first jig can comprise a flexible or substantially non-rigid material (or portions thereof) which allows the first jig to deform, flex and/or "snap fit" around some or all of a proximal tibial head. In various examples, the inner surface of the first jig can be designed to accommodate, reference, and/or avoid various surface features on the tibia, such as the presence of osteophytes or other anatomical projections on the tibia. In various alternative examples, the first jig can include one or more features that align the jig using the aforementioned osteophytes or other anatomical projections.

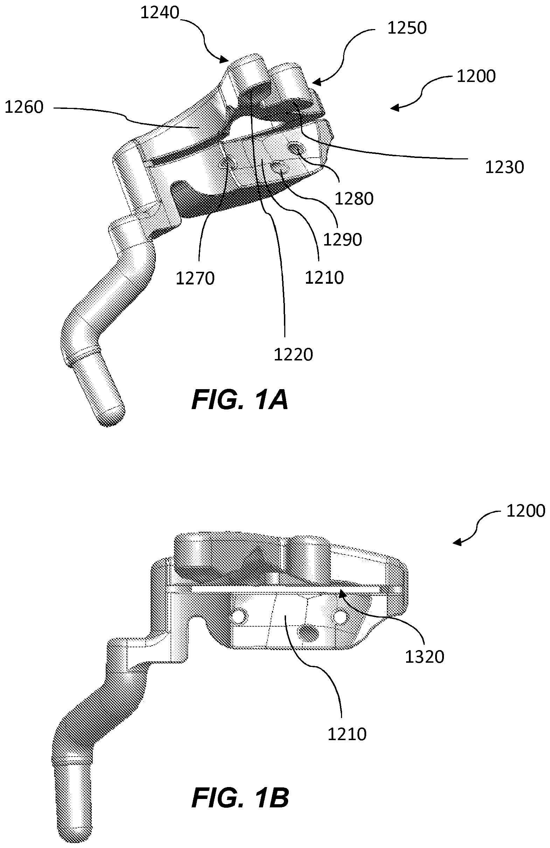

FIGs. 1A and B depict views of a first tibial jig 1200, which includes one or more surfaces designed and/or selected to accommodate and/or conform to various anatomical features and/or surfaces of the underlying tibial anatomy. The first jig 1200 can include a generally posterior-oriented or facing surface 1210 and one or more caudad-oriented or capping surfaces 1220 and 1230 formed on projections 1240 and 1250 that extend from an upper portion 1260 of the first jig 1200. In use, the surface 1210 can be shaped to conform to an anterior-facing portion of the tibial head (not shown), with the capping surfaces 1220 and 1230 conforming to corresponding subchondral bone surfaces of the proximal tibia (not shown). When properly positioned on the tibia, this arrangement and placement can result in alignment of the first jig 1200 in a known position and/or orientation. Additionally, first jig 1200 can include one or more slots 1270, 1280 and 1290, which can be configured to guide insertion of surgical instruments (e.g., one or more alignment pins or wires) into the tibia to align and/or secure the jig (or various other tools) to the tibia.

In various examples, anatomical imaging data can be used to design and arrange cap surfaces 1220 and 1230 of first jig 1200 to rest against corresponding portions of subchondral bone surfaces on the proximal tibial head. Subchondral bone surfaces may be utilized to reference the jig, because such surfaces can be relatively easily imaged using X-ray or CT scan technology and segmented by an automated system. In contrast, softer tissues such as articular cartilage may be more difficult to image and/or segment by an operator and/or automated system, but where imaging such as MRI is utilized, the use of such surfaces for alignment is contemplated in various alternative examples.

Since the anterior face of the tibia typically does not include significant amounts of articular cartilage, this surface of the tibia can often be readily used as a reference surface. The articulating superior (or cephalad facing) surface of the tibia can be almost entirely covered with cartilage (at least in its healthy state). Where such subchondral bone surfaces are covered by articular cartilage, it may become desirable and/or necessary to remove various overlying articular cartilage (as well as any interfering osteophytes and/or other anatomical structures that may not be accounted for in the initial jig design) to facilitate the ultimate placement of first jig 1200.

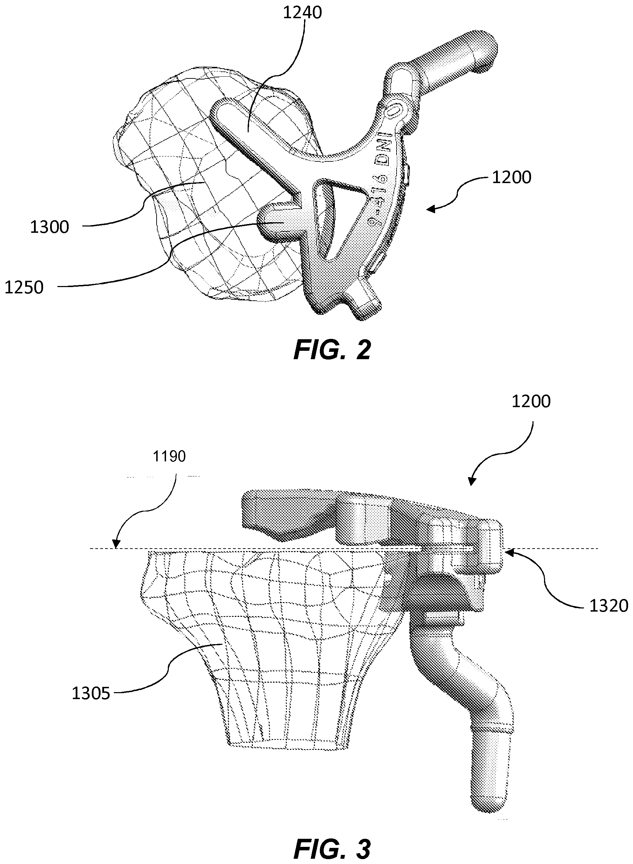

To effectuate such placement and alignment using subchondral bone, in some examples, first jig 1200 can initially be placed adjacent tibial anterior surface 1300 in a desired orientation (see FIG. 2), and the outline of the projections 1240 and 1250 and/or other jig features adjacent the anterior tibial plateau can be marked on the tibia using a surgical marker, and the jig removed. Once the jig is removed, the surgeon can remove articular cartilage and any residual or interfering tissue encompassing and/or anterior to the marked portion (e.g., using a curette, scalpel or other surgical tool), exposing the underlying subchondral bone of the tibia. After relevant portions of the subchondral bone have been exposed in a desired manner, jig 1200 can be repositioned on the tibia, with the relevant matching and/or conforming facing surface 1210 and capping surfaces 1220 and 1230 in substantial contact with the tibia.

According to the invention, jig 1200 includes one or more patient-adapted surfaces for engaging a portion of the patient's skin. As illustrated in FIG. 4, jig 1200 can include an arm 1110 that has at least one surface with a shape based, at least in part, on patient-specific information regarding the shape of a corresponding portion of a skin surface 1120 of the patient that is adjacent to the knee joint. For example, as depicted in FIGs. 4, 5A, and 5B, in some embodiments, arm 1110 can be provided with a substantially posterior-facing surface 1130 that is shaped to substantially conform to a portion of a substantially anterior-facing skin surface 1120 that is distal or inferior to the incision through which surgical access to the proximal tibia is provided. In some embodiments, jig 1200 and/or arm 1110 can be configured such that when skin-referencing surface 1130 is properly engaged and aligned with the corresponding portion of skin surface 1120, jig 1200 (including one or more of the guide slots (e.g., 1270, 1280, 1290, 1320)) is aligned in a predetermined position and orientation relative to the patient's proximal tibia. In this manner, skin referencing surface 1130 can provide a surface for alignment and/or stabilization of jig 1200, in addition to one or more of the patient-adapted surfaces 1210, 1220, and/or 1230, described above. Accordingly, when a surgeon positions jig 1200 during a procedure, skin-referencing surface 1130 can provide improved identification of the proper position and/or alignment of jig 1200 on the patient's anatomy, an additional reference for proper alignment of the jig with respect to one or more anatomical and/or mechanical axis (e.g., in the sagittal plane and/or the coronal plane), and/or enhanced stability of jig 1200, which may ease use of surgical instruments with the various guide slots.

A patient-adapted skin-referencing surface may be utilized with a jig in a variety of configurations. In some embodiments, a patient-adapted skin-referencing surface may be integrally formed in the body of a jig. For example, a tibial jig similar to jig 1200 described above, which includes one or more patient-adapted surfaces configured to engage surfaces of the tibia (e.g., 1210, 1220, 1230) and one or more slots (e.g., 1270, 1280, 1290, 1320) configured to guide surgical instruments in predetermined positions and/or orientations, can include a patient-adapted skin-referencing surface. For example, in some embodiments, the tibial jig can include an arm 1110, integrally formed with and extending from the jig and having a patient-adapted surface configured to engage a portion of a skin surface. Additionally or alternatively, a patient-adapted surface configured to engage a portion of a skin surface may be included as an integral surface of any portion of the tibial jig.

Additionally or alternatively, a patient-adapted skin-referencing surface may be formed as a modular component that is releasably attachable to a jig. For example, as depicted in FIGs. 5A and 5B, arm 1110 can include a mating feature 1140 configured to releasably couple arm 1110 to a corresponding mating feature 1150 of jig 1200 in a predetermined position and/or orientation. Mating features 1140 and 1150 can comprise any of various mating and/or coupling structures known in the art, such as, for example, a dovetail coupling, a threated coupling, a cantilevered snap-fit coupling, and/or generally, a female feature having a recess or opening dimensioned to receive a corresponding male feature, resulting in a desired coupling fit (e.g., mechanical, interference, transition, or clearance). In some embodiments, mating feature 1150 may be located on or near a projecting arm 1345. Projecting arm 1345 may be configured to extend generally through the surgical excision from a portion of jig 1200 positioned on a patient's tibia. As discussed further below, in some embodiments, projecting arm 1345 may include a fitting 1340 configured to be coupled to an alignment rod extension 1330. In some embodiments, arm 1110 and/or jig 1200 may be provided with reference markings to guide proper attachment of arm 1110 to jig 1200. In some embodiments of a modular configuration, arm 1110 may be included in a surgical kit as already coupled to jig 1200. Providing the skin-referencing surface as either integrally formed with jig 1200 or modular but pre-coupled to jig 1200 may advantageously eliminate an extra surgical step of connecting arm 1110 to jig 1200.

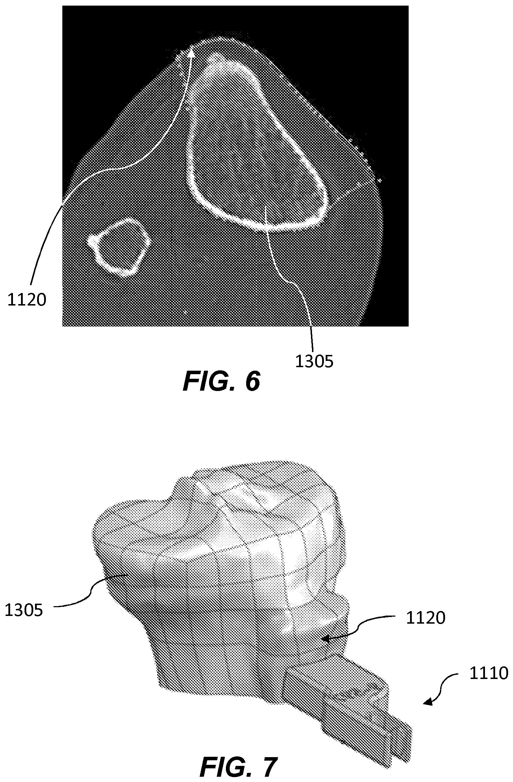

In various examples, a patient-adapted skin-referencing surface may be designed utilizing one or more of the processes described herein and/or in U.S. Patent Publication No. 2012-0209394 for producing patient-adapted features corresponding to other tissue types (e.g., bone, cartilage). For example, patient-specific imaging data obtained regarding a patient's knee joint may include information regarding a skin surface adjacent to the knee joint. FIG. 6 depicts exemplary imaging information associated with a portion of the patient's tibia 1305. In addition to information regarding the shape and location of the tibia 1305, the same imaging information can be used to identify a surface outline of a skin surface 1120 adjacent to the tibia. As shown, the distinctive transition in color intensity or grayscale at the skin surface 1120 can be used to identify pixels, voxels, corresponding data points, a continuous line, and/or surface data indicative of at least a portion of the shape and/or the location of the skin surface 1120 relative to other anatomical features (e.g., the tibia). Accordingly, such information can be derived from and utilized in processes, such as, for example, those described in detail in U.S. Patent Publication No. 2012-0209394 to create a model of a portion of the skin surface 1120. In some examples, the model of the portion of the skin surface 1120 may be included in and/or combined with a model of the tibia 1305, as shown in FIG. 7. In various examples, the shape for a patient-adapted skin-referencing surface can be derived based, at least in part, on a model of the portion of the skin surface 1120. Accordingly, in some examples, the shape of a patient-adapted skin-referencing surface may be designed to conform to the shape of a corresponding portion of the patient's skin in one or more planes (e.g., sagittal, coronal, transverse).

A patient-adapted skin-referencing surface may be designed to engage a particular skin surface at a variety of different anatomical locations, depending on factors such as the particular tissue for which it is being used to prepare (e.g., knee, proximal tibia, distal femur, hip, shoulder, ankle), the surgical plan, location of skin captured in imagining acquired for designing the surgical system, and/or characteristics of skin surfaces and underlying soft tissue adjacent to the surgical site. For example, in the case of a skin-referencing surface for tibial jig 1200, a patient-adapted skin-referencing surface may be designed to engage a portion of the skin surface residing substantially anterior and adjacent to the shaft of the tibia and distal (inferior) to the incision that provides surgical access to the tibia. In some examples, the skin-referencing surface may be designed to be substantially centered in a coronal plane with respect to a ridge or convexity of the tibial shaft. Such a configuration may improve stability and/or registration of the skin-referencing surface when engaging the corresponding referenced skin surface. Accordingly, while in some examples, a skin-referencing surface may be substantially aligned in a coronal plane with a mechanical axis of the tibia and/or knee joint, in other examples, a skin-referencing surface may be medially or laterally displaced relative to a mechanical axis. In some instances, a skin surface located substantially anterior to the tibial shaft may provide a better (e.g., more stable) reference location than others because a relatively minimal amount of soft tissue may be found between the skin and the underlying bone at this location, thereby potentially reducing the amount of compression and/or movement of the skin surface relative to the tibia.

In various examples, identifying/selecting a portion of the skin to be referenced for a tibial jig may include selecting a location that is distal to the patellar ligament insertion and/or the tibial tuberosity. In some examples, such locations may be identified based on patient-specific information and/or estimated based on patient-specific information and/or general anatomical information. Additionally or alternatively, in some examples, the distalmost portion of skin included in available imaging information of the knee joint may be selected as a portion of skin to be referenced. In some cases, referencing a skin location that is relatively further away from the tissue to be treated may provide improved overall stability and/or accuracy of alignment of the jig. One advantage of utilizing various examples of skin-referencing surfaces disclosed herein with a jig can be referencing general anatomical locations that would otherwise be undesirable and/or impossible to reference using surfaces below the skin (e.g., bone surfaces, cartilage surfaces) from the surgical site due to the increased length of incisions and/or general invasiveness of the procedure necessary to provide access to such locations.

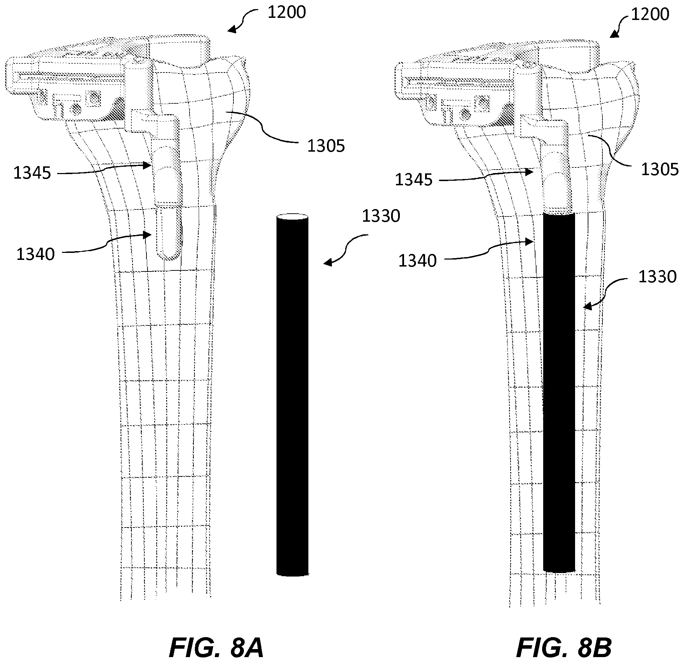

In some examples, the surgeon may choose to assess the alignment of tibial jig 1200 relative to an axis of the tibia 1305 and/or lower extremity. In some examples, this can include connecting an alignment rod extension 1330 to a corresponding fitting 1340 on a projecting arm 1345 of jig 1200 (see FIGs. 8A and 8B). The surgeon can then visually and/or tactilely assess the rod extension 1330 relative to the tibia 1305, lower extremity, ankle, foot and/or other anatomical structures (e.g., center of the malleoli) to determine the medial/lateral and/or anterior/posterior tilt or slope(s) of the jig. Where there is significant variation between the extension rod's position/orientation relevant to the target anatomy and the extension rod's expected position/orientation, the discrepancy may indicate, for example, that (1) the jig is positioned incorrectly and/or is not appropriate for the patient, (2) the anatomy was imaged incorrectly or the anatomical information and/or jig design did not properly accommodate some feature of the patient's anatomy, (3) structures interfering with jig placement (e.g., articular cartilage and/or osteophytes) may remain on the tibial surface, or (4) the variation is intended by the surgeon (e.g., to alter the valgus/varus tilt of the resulting knee implant). In various circumstances, the surgeon may choose to use the extension to realign the jig prior to placement of alignment and/or securement pins, or the jig may be removed and the relevant anatomy assessed further (and potentially further modified) by the surgeon. Once a desired alignment has been obtained, or when the alignment of the jig has been verified to the surgeon's satisfaction, rod extension 1330 can be removed from jig 1200.

In some examples, once jig 1200 has been properly positioned relative to the tibia, the surgeon can drill through openings 1270 and 1280 in the jig 1200 and introduce one or more alignment or anchoring pins into and through the jig, desirably anchoring the pins in the tibia. These pins can be introduced in a relatively parallel fashion, which can allow the jig 1200 to be removed from the tibia and replaced with subsequent jigs, sliding the new jig along the parallel alignment pins and, if desired, into contact with the tibia. Additionally or alternatively, once jig 1200 has been properly positioned relative to the tibia, the surgeon can saw through slot 1320, which can be configured to guide a surgeon's resection of some or all of the proximal head of the tibia along a predetermined cut plane 1190, as depicted in FIG. 3. In various examples and at various points in the surgery (e.g., prior to a resection cut), the surgeon may choose to drill through a securement or stabilization opening 1290 in the jig and introduce a securement pin through the opening and into the tibia. The securement pin can be non-parallel to the alignment pins, and when introduced through the jig 1200 and into the tibia can result in the jig 1200 being substantially fixed and secured to the tibia. If subsequent removal of the jig 1200 is desired, the securement pin can be withdrawn from the tibia, and the jig removed by sliding it along the alignment pins and away from the tibia. If desired, a subsequent jig can be introduced onto the alignment pins, slid towards the tibia, and secured to the tibia using a similar securement pin arrangement.

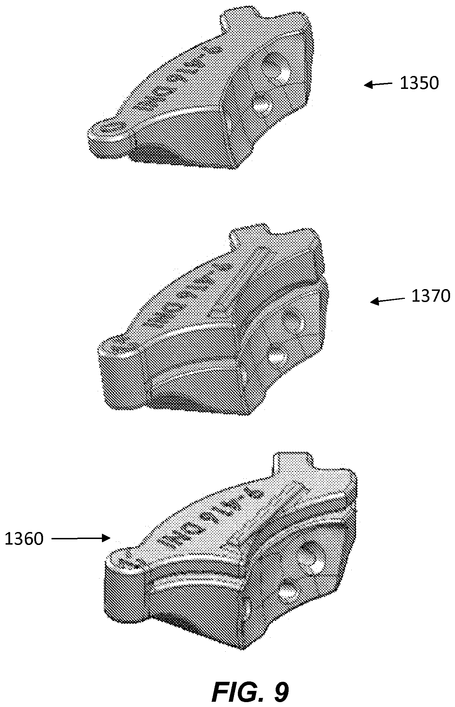

In some examples, exemplary additional jigs that may be provided can include jigs with guide slots configured to guide resection of the tibia at different depths or heights. For example, as depicted in FIG. 9, such jigs can include an uncaptured 0 mm depth jig 1350, a captured -2 mm 1360, and a captured +2 mm jig 1370. In use the surgeon can utilize first jig 1200 as previously described to position and implant alignment pins, and then remove the first jig 1200 and replace it with an alternative tibial jig 1350, 1360 or 1370. Depending upon the surgeon's assessment of the patient anatomy, as well as any desired joint balancing using additional surgical tools (e.g., trials implants, balancing chips, assessment probes), the surgeon may desire to modify the pre-planned tibial preparation procedure to increase, decrease and/or alter the surgical resection(s). For example, if the surgeon wishes to increase the depth of the surgical resection by an additional 2 mm beyond the pre-planned depth, the surgeon may choose to utilize the +2 mm captured tibial jig 1370. Alternatively, the surgeon may desire to decrease the depth of the surgical resection by an additional 2 mm less than the pre-planned depth, and thus may choose to employ the -2 mm captured tibial jig 1370. In a similar manner, if the surgeon prefers to utilize an uncaptured guide surface instead of the capture guide surface of the first jig 1200, the surgeon may choose to employ the 0 mm uncaptured tibial jig 1350, which the surgeon may feel allows for greater flexibility in tibial preparation during the surgical procedure.

In various alternative examples, tibial jigs may be provided to alter and/or modify the pre-planned surgical procedures in a variety of ways. For example, a single tibial jig can be provided that accommodates a more complex tibial resection, such as medial and lateral resections separated by a step-cut (e.g., vertical and/or angled) surface. In alternative examples, a complex tibial surface may be created using a plurality of tibial jigs, such as a first jig that incorporates one or more first cutting or guiding feature(s) (e.g., a lateral, substantially horizontal resection combined with a centrally-located step cut), in combination with a second tibial jig that incorporates one or more second cutting or guiding feature(s) (e.g., a medial, substantially tilted resection combined with a more centrally-located step vertical cut that desirably creates a complex step-cut geometry). The use of multiple tibial jigs, in combination with alignment and securement pins as described herein, can allow for significant improvement in the preparation of the tibial anatomical support surface, even in conditions of reduced visibility (e.g., minimally invasive procedures).

The various additional tibial jig options described herein can provide the surgeon with increased flexibility to assess the tibial anatomy and surgically resect various portions of the tibial head based on intraoperative observations. For example, if a patient has experienced significant or excessive wear on a medial tibial plateau, such as where the patient's varus deformity is greater than 10 degrees, this deformity might not be readily apparent from an initial anatomical assessment and/or non-invasive imaging study. Where tibial jigs have been created without accounting for such a deformity, the resulting planned resection could potentially result in an aggressive lateral resection (e.g., potentially greater than 7 mm), which may be undesirable. Where such a deformity becomes apparent to the surgeon during the procedure (e.g., by direct visualization and/or removal of the overlying articular cartilage, or misalignment of the previously described alignment rod extension), or where the imaging study indicates a potential for the existence of such a deformity (e.g., a greater than 7 mm depth resection identified on the pre-operative surgical plan), the surgeon may choose to utilize the -2 mm tibial cut jig for a primary cut on the tibia, which can desirably raise the tibial resection plane to a depth of 0 mm below the lowest point on the medial tibial plateau.

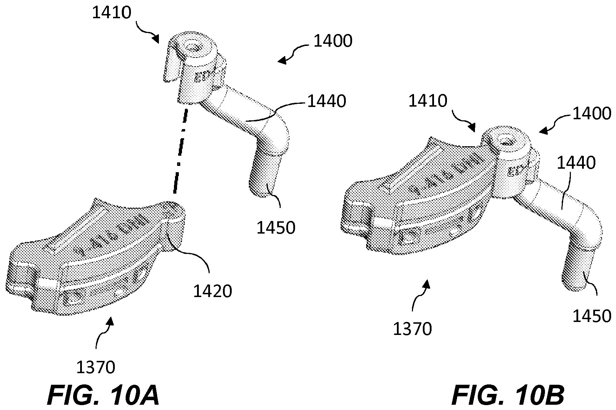

FIGs. 10A and B depict one exemplary example of a modular alignment adapter 1400 that can be used in combination with various of the jigs disclosed herein (e.g., those depicted in FIG. 9) and a rod extension (e.g., as depicted in FIGs. 8A and B) and/or a skin-referencing arm 1110 (e.g., as depicted in FIGs. 5A and 5B). Adapter 1400 can include a docking cap 1410, which desirably fits over and engages with a corresponding protrusion 1420 on the jig. The adapter 1400 further includes an extension arm 1440 that extends from the docking cap 1410 to a fitting 1450. Fitting 1450 can be sized and configured to engage an alignment rod extension (not shown) in a manner similar to that described and depicted in FIGs. 8A and B. Additionally or alternatively, extension arm 1440 can include a mating feature configured to engage a mating feature of arm 1110, as depicted in FIGs. 4, 5A, and 5B and described above. In use, the adapter 1400 and associated extension rod and/or arm 1110 can be secured to the tibial jig and utilized to stabilize the jig and/or verify alignment of the jig relative to a desired target anatomy.

In various examples described herein, the knee rotation axis may be derived from various patient-specific data or combinations of patient-specific data, including from the imaged and/or derived and/or normalized medial/lateral J-curve data (e.g., medial or lateral or combinations of both). Moreover, the knee rotation axis can be derived from J-curve data from the femur and/or tibia or combinations thereof. Additionally or alternatively, the knee rotation axis may be derived from various other axes, including the transepicondylar axis and/or the posterior condylar axis. In a similar manner, the implant motion (e.g., flexion, extension, translation and/or rotation) can be derived from medial and/or lateral tibial slope of the patient and/or from an engineered design and/or combinations thereof, as well as from various combinations of the knee rotation axis and implant motion.

While various examples provided above are generally described with respect to treatment of a knee joint, various aspects and embodiments disclosed herein can be applied to treatment of any anatomical feature and/or joint. For example, patient-adapted skin-referencing surfaces may be designed to engage particular skin surfaces at a variety of different anatomical locations and for use in a variety of different applications. Patient-adapted skin-referencing surfaces may be designed for use with surgical guides for treatments such as, for example, total joint repair, partial joint repair, or ligament repair and in areas such as, for example, the hip, shoulder, or ankle. In various embodiments, patient-adapted skin-referencing surfaces may be designed to reference skin surfaces adjacent to the location of treatment (e.g., hip, shoulder, ankle) and/or to reference skin surfaces adjacent to anatomical features removed or distanced from the location of treatment. By way of example, a guide for use in hip replacements could be provided with a patient-adapted skin-referencing surface designed to reference a skin surface adjacent to the tibia. Such a guide could be used to help set or measure the rotational angle or anteversion of a femoral component being implanted in the hip (currently, surgeons often flex the knee 90 degrees and use the tibial axis to gauge the femoral component rotation in hip replacement procedures). Similarly, a guide referencing skin adjacent to the forearm could be provided for use in shoulder replacements and a guide configured to reference skin over the foot could be provided for use in knee replacements. As another example, surgical boots or extremity holders used during surgical procedures to rigidly hold a patient's extremity (e.g., leg, arm) could be provided with one or more patient-adapted skin-referencing surfaces to optimize the fit of the holder for the particular patient.

The various descriptions contained herein are merely exemplary in nature and, thus, it will be understood that the scope of the invention is limited only by the appended claims.

## Claims

1. A guide (1200) for treating a knee joint of a patient, the guide comprising:
a first portion (1110), the first portion including at least one surface (1130) having a shape based, at least in part, on patient-specific information regarding a portion of a skin surface of the patient adjacent to the joint, so as to conform to said portion of a skin surface; and
a second portion, the second portion comprising:
a first surface (1210) shaped, at least in part, to conform to an anterior-facing portion of the tibial head of the patient;
a second surface (1220, 1230) shaped, at least in part, to conform to the corresponding subchondral bone surface of the proximal tibia of the patient; and
a guiding formation (1320) configured to guide a surgical instrument, the guiding formation having a predetermined position and orientation with respect to the at least one surface of the first portion and the first and second surface of the second portion.

2. The guide of claim 1, wherein the first portion is releasably attached to the second portion.

3. The guide of claim 1, wherein the first portion is integral with the second portion.

4. The guide of claim 1, wherein the portion of the skin surface of the patient is a portion located substantially anterior and adjacent to a portion of a shaft of the tibia.

5. The guide of claim 1, wherein the second portion further comprises one or more surfaces having a shape based, at least in part, on a shape of a portion of a medial and/or lateral plateau of the tibia.

6. The guide of claim 1, wherein the second portion comprises a second surface having a shape based, at least in part, on a shape of a portion of a medial plateau of the tibia and further comprises a third surface having a shape based, at least in part, on a shape of a portion of a lateral plateau of the tibia.

7. A system for treating a knee joint of a patient, the system comprising:
a first guide tool (1110), the first guide tool comprising:
at least one surface (1130) having a shape based, at least in part, on patient-specific information regarding a portion of a skin surface of the patient adjacent to the joint, so as to conform to said portion of a skin surface; and
a mating feature (1140);
a surgical instrument guide (1200), the surgical instrument guide comprising:
a first surface (1210) shaped, at least in part, to conform to an anterior-facing portion of the tibial head of the patient;
a second surface (1220, 1230) shaped, at least in part, to conform to the corresponding subchondral bone surface of the proximal tibia of the patient;
a guiding formation (1320) configured to guide a surgical instrument; and
a mating feature (1150), wherein the mating feature of first guide tool is configured to engage the mating feature of the surgical instrument guide and couple the first guide tool to the surgical instrument guide in a predetermined position and orientation,
wherein the guiding formation has a predetermined position and orientation with respect to the at least one surface of the first guide tool and the first and second surface of the surgical instrument guide, when the first guide tool is coupled to the surgical instrument guide via the mating features.

8. A system for treating a joint of a patient according to claim 7, wherein the system further comprises an implant component to be implanted in the joint.

9. The system of claim 8, wherein the implant component includes at least one bone-facing surface and wherein the guiding formation is configured to guide a saw along a predetermined cutting plane such that a resected surface of the joint is formed and configured to support the at least one bone-facing surface of the implant.

10. The system of claim 8, wherein the implant component includes at least one joint-facing surface having a shape based, at least in part, on patient-specific information regarding the joint.

11. The system of claim 7 or of claim 8, wherein the portion of the skin surface of the patient is a portion located substantially anterior and adjacent to a portion of a shaft of the tibia.

12. The system of claim 7 or of claim 8, further comprising an alignment rod configured to be releasably coupled to the surgical instrument guide in a predetermined position and orientation.

13. The guide of claim 1, the system of claim 7, or the system of claim 8, wherein the patient-specific information regarding the skin surface comprises information regarding a shape of the skin surface in at least one plane.

## Patentansprüche

1. Einen Guide (1200) zur Behandlung eines Kniegelenks eines Patienten, wobei der Guide umfasst:
einen ersten Abschnitt (1110), wobei der erste Abschnitt mindestens eine Oberfläche (1130) umfasst, die eine Form hat, die zumindest teilweise auf Patienten-spezifischen Informationen hinsichtlich eines Abschnitts einer Hautoberfläche des Patienten angrenzend an das Gelenk basiert, so dass sie jenem Abschnitt der Hautoberfläche entspricht; und
einen zweiten Abschnitt, wobei der zweite Abschnitt umfasst:
eine erste Oberfläche (1210), die zumindest teilweise so geformt ist, dass sie einem nach vorne weisenden Abschnitt des Tibiakopfes des Patienten entspricht;
eine zweite Oberfläche (1220, 1230), die zumindest teilweise so geformt ist, dass sie der entsprechenden subchondralen Knochenoberfläche der proximalen Tibia des Patienten entspricht; und
eine Guide-Formation (1320), die konfiguriert ist, um ein chirurgisches Instrument zu führen, wobei die Guide-Formation eine vorbestimmte Position und Orientierung in Bezug auf die mindestens eine Oberfläche des ersten Abschnitts und die erste und zweite Oberfläche des zweiten Abschnitts aufweist.

2. Der Guide nach Anspruch 1, wobei der erste Abschnitt lösbar an dem zweiten Abschnitt angebracht ist.

3. Der Guide nach Anspruch 1, wobei der erste Abschnitt integral mit dem zweiten Abschnitt ist.

4. Der Guide nach Anspruch 1, wobei der Abschnitt der Hautoberfläche des Patienten ein Abschnitt ist, der im Wesentlichen anterior und benachbart zu einem Abschnitt eines Schafts der Tibia angeordnet ist.

5. Der Guide nach Anspruch 1, wobei der zweite Abschnitt ferner eine oder mehrere Oberflächen umfasst, die eine Form haben, die zumindest teilweise auf der Form eines Abschnitts eines medialen und / oder lateralen Plateaus der Tibia basiert.

6. Der Guide nach Anspruch 1, wobei der zweite Abschnitt eine zweite Oberfläche umfasst, die eine Form hat, die zumindest teilweise auf einer Form eines Abschnitts eines medialen Plateaus der Tibia basiert, und ferner eine dritte Oberfläche umfasst, die eine Form hat, die zumindest teilweise auf einer Form eines Abschnitts eines lateralen Plateaus der Tibia basiert.

7. Ein System zur Behandlung eines Kniegelenks eines Patienten, wobei das System umfasst:
ein erstes Guide-Tool (1110), wobei das erste Guide-Tool umfasst:
mindestens eine Oberfläche (1130), die eine Form hat, die zumindest teilweise auf Patienten-spezifischen Informationen hinsichtlich eines Abschnitts einer Hautoberfläche eines Patienten angrenzend an das Gelenk basiert, so dass sie einem Abschnitt einer Hautoberfläche entspricht; und
ein Verbindungsmerkmal (1140);
einen chirurgischen Instrumenten-Guide, wobei der chirurgische Instrumenten-Guide umfasst:
eine erste Oberfläche (1210), die zumindest teilweise so geformt ist, dass sie einem nach vorne weisenden Abschnitt des Tibiakopfes des Patienten entspricht;
eine zweite Oberfläche (1220, 1230), die zumindest teilweise so geformt ist, dass sie der entsprechenden subchondralen Knochenoberfläche der proximalen Tibia des Patienten entspricht;
eine Guide-Formation (1320), die konfiguriert ist, um ein chirurgisches Instrument zu führen; und
ein Verbindungsmerkmal (1150), wobei das Verbindungsmerkmal des ersten Guide-Tools konfiguriert ist, um in das Verbindungsmerkmal des chirurgischen Instrumenten-Guides einzurasten und das erste Guide-Tool in einer vorbestimmten Position und Orientierung mit dem chirurgischen Instrumenten-Guide zu koppeln,
wobei die Guideformation eine vorbestimmte Position und Orientierung in Bezug auf die mindestens eine Oberfläche des ersten Guide-Tools und die erste und zweite Oberfläche des chirurgischen Instrumenten-Guides aufweist, wenn das erste Guide-Tool über die Verbindungsmerkmale mit dem chirurgische Instrumenten-Guide gekoppelt ist.

8. Ein System zur Behandlung eines Gelenks eines Patienten nach Anspruch 7, wobei das System ferner eine Implantatkomponente umfasst, die in das Gelenk implantiert werden soll.

9. Das System nach Anspruch 8, wobei die Implantatkomponente mindestens eine dem Knochen zugewandte Oberfläche umfasst und wobei die Guideformation konfiguriert ist, um eine Säge entlang einer vorbestimmten Schnittebene zu führen, so dass eine resezierte Oberfläche des Gelenks gebildet und konfiguriert wird, um die mindestens eine dem Knochen zugewandte Oberfläche des Implantats zu unterstützen.

10. Das System nach Anspruch 8, wobei die Implantatkomponente mindestens eine dem Gelenk zugewandte Oberfläche umfasst, die eine Form hat, die zumindest teilweise auf Patienten-spezifischen Informationen in Bezug auf das Gelenk basiert.

11. Das System nach Anspruch 7 oder Anspruch 8, wobei der Abschnitt der Hautoberfläche des Patienten ein Abschnitt ist, der im Wesentlichen anterior und angrenzend zu einem Abschnitt eines Schafts der Tibia angeordnet ist.

12. Das System nach Anspruch 7 oder Anspruch 8, das ferner einen Ausrichtungsstab umfasst, der so konfiguriert ist, dass er in einer vorbestimmten Position und Orientierung lösbar mit dem chirurgischen Instrumenten-Guide gekoppelt ist.

13. Der Guide nach Anspruch 1, das System nach Anspruch 7 oder das System nach Anspruch 8, wobei die Patienten-spezifischen Informationen bezüglich der Hautoberfläche Informationen bezüglich einer Form der Hautoberfläche in mindestens einer Ebene umfassen.

## Revendications

1. Guide (1200) pour traiter une articulation de genou d'un patient, le guide comprenant :
une première portion (1110), la première portion incluant au moins une surface (1130) ayant une forme basée, au moins en partie, sur des informations spécifiques au patient concernant une portion d'une surface de peau du patient adjacente à l'articulation, de manière à se conformer à ladite portion d'une surface de peau ;
une seconde portion, la seconde portion comprenant :
une première surface (1210) mise en forme, au moins en partie, pour se conformer à une portion tournée vers la zone antérieure de la tête tibiale du patient ;
une deuxième surface (1220, 1230) mise en forme, au moins en partie, pour se conformer à la surface osseuse sous-chondrale correspondante du tibia proximal du patient ; et
une formation de guidage (1320) configurée pour guider un instrument chirurgical, la formation de guidage ayant une position et une orientation prédéterminées par rapport à l'au moins une surface de la première portion et aux première et deuxième surfaces de la seconde portion.

2. Guide selon la revendication 1, dans lequel la première portion est fixée de manière libérable à la seconde portion.

3. Guide selon la revendication 1, dans lequel la première portion fait corps avec la seconde portion.

4. Guide selon la revendication 1, dans lequel la portion de la surface de peau du patient est une portion située de manière sensiblement antérieure et adjacente à une portion d'une diaphyse tibiale.

5. Guide selon la revendication 1, dans lequel la seconde portion comprend en outre une ou plusieurs surfaces ayant une forme basée, au moins en partie, sur une forme d'une portion d'un plateau médial et/ou latéral du tibia.

6. Guide selon la revendication 1, dans lequel la seconde portion comprend une deuxième surface ayant une forme basée, au moins en partie, sur une forme d'une portion d'un plateau médial du tibia et comprend en outre une troisième surface ayant une forme basée, au moins en partie, sur une forme d'une portion d'un plateau latéral du tibia.

7. Système pour traiter une articulation de genou d'un patient, le système comprenant :
un premier outil de guidage (1110), le premier outil de guidage comprenant :
au moins une surface (1130) ayant une forme basée, au moins en partie, sur des informations spécifiques au patient concernant une portion d'une surface de peau du patient adjacente à l'articulation, de manière à se conformer à ladite portion d'une surface de peau ;
un accessoire d'appariement (1140) ;
un guide d'instrument chirurgical (1200), le guide d'instrument chirurgical comprenant :
une première surface (1210) mise en forme, au moins en partie, pour se conformer à une portion tournée vers la zone antérieure de la tête tibiale du patient ;
une deuxième surface (1220, 1230) mise en forme, au moins en partie, pour se conformer à la surface osseuse sous-chondrale correspondante du tibia proximal du patient ;
une formation de guidage (1320) configurée pour guider un instrument chirurgical ; et
un accessoire d'appariement (1150),
dans lequel l'accessoire d'appariement du premier outil de guidage est configuré pour entrer en prise avec l'accessoire d'appariement du guide d'instrument chirurgical et accoupler le premier outil de guidage au guide d'instrument chirurgical dans une position et une orientation prédéterminées,
dans lequel la formation de guidage a une position et une orientation prédéterminées par rapport à l'au moins une surface du premier outil de guidage et aux première et deuxième surfaces du guide d'instrument chirurgical, quand le premier outil de guidage est accouplé au guide d'instrument chirurgical via les accessoires d'appariement.

8. Système pour traiter une articulation d'un patient selon la revendication 7, dans lequel le système comprend en outre un composant d'implant à implanter dans l'articulation.

9. Système selon la revendication 8, dans lequel le composant d'implant inclut au moins une surface tournée vers l'os et dans lequel la formation de guidage est configurée pour guider une scie le long d'un plan de coupe prédéterminé de telle sorte qu'une surface reséquée de l'articulation est formée et configurée pour supporter l'au moins une surface tournée vers l'os de l'implant.

10. Système selon la revendication 8, dans lequel le composant d'implant inclut au moins une surface tournée vers l'articulation ayant une forme basée, au moins en partie, sur des informations spécifiques au patient concernant l'articulation.

11. Système selon la revendication 7 ou la revendication 8, dans lequel la portion de la surface de peau du patient est une portion située de manière sensiblement antérieure et adjacente à une portion d'une diaphyse tibiale.

12. Système selon la revendication 7 ou la revendication 8, comprenant en outre une tige d'alignement configurée pour être accouplée de manière libérable au guide d'instrument chirurgical dans une position et une orientation prédéterminées.

13. Système selon la revendication 1, système selon la revendication 7, ou système selon la revendication 8, dans lequel les informations spécifiques au patient concernant la surface de peau comprennent des informations concernant une forme de la surface de peau dans au moins un plan.
